# EUROPEAN PATENT APPLICATION

(11) **EP 4 134 106 A2**
(43) Date of publication of application: **15.02.2023**
(21) Application number: 22175204.1
(22) Date of filing: 24.05.2022
(51) Int. Cl.: A61L 9/20, B60H 3/00

(54) **UVC LED DISINFECTING DEVICE**

(30) Priority: 25.05.2021 US 202117329595; 07.09.2021 US 202117468226
(71) Applicant: Wu, Bor-Jen, New Taipei City 224019 (TW); Tsen, Chia-Bin, New Taipei City 234022 (TW); Wang, Yu-Yen, Taoyuan City 320073 (TW); Hsu, Hui-Chi, New Taipei City 234022 (TW); Tsai, Hsien-Hsin, Taipei City 104099 (TW)
(72) Inventor: Wu, Bor-Jen, New Taipei City 224019 (TW); Tsen, Chia-Bin, New Taipei City 234022 (TW); Wang, Yu-Yen, Taoyuan City 320073 (TW); Hsu, Hui-Chi, New Taipei City 234022 (TW); Tsai, Hsien-Hsin, Taipei City 104099 (TW)
(74) Representative: DREISS Patentanwälte PartG mbB

(57) **Abstract**

This invention provides an air disinfecting device for disinfecting or decontaminate air, which comprises a first metal plate (44) and a second metal plate (42) opposite to the first metal plate (44), a UVC LED (26) mounted on the first metal plate (44), and a power supply providing power to the UVC LED (26). The second metal plate (42) has an area large enough such that lights emitted from the UVC LED (26) will be enclosed inside the air disinfection device.

## Description

### CLAIM OF PRIORITY

This application is a Continuation-in-part of U.S. application No. 17/ 329,595 entitled to inventors filed May 25th, 2021 and entitled "UVC LED Disinfecting Device", the entire disclosures of which are incorporated herein by reference.

### FIELD OF THE INVENTION

The invention relates to a sterilization device, and more particularly to a UVC LED disinfecting device.

### BACKGROUND OF THE INVENTION

UVC(ultraviolet-C) tubes or bulbs have been widely used in conventional disinfecting devices, such as US patent number 7,175,814 by Dionisio, titled as "AIR DISINFECTING SYSTEM AND CARTRIDGE DEVICE CONTAINING ULTRAVIOLET LIGHT". Dionisio teaches a mobile air disinfection device which includes a UV bulb, HEPA or carbon filter, a fan for active air flow, a battery, and a LED indicator for battery life.

Both the UV light bulb and the fan for air flow are high power consumption devices, and thus would not be suitable for long duration mobile applications. Furthermore, the UV light bulb is too bulky to many other mobile applications.

Thus, an invention is necessary to solve the issues mentioned above.

### BRIEF SUMMARY OF THE INVENTION

The objective of this invention is to provide an air disinfection device which can eliminate bacteria, viruses, even foul odors.

It is an objective of this invention to provide an air disinfecting device within air circulation passage way of appliances, such as refrigerators, air conditioners, air purifiers, dehumidifiers, or humidifiers.

It is an objective of this invention to provide an air disinfecting device inside an air circulation system of buildings for habitat or living, such as residential houses, hospitals, theaters, malls, stadiums, cleanrooms, factories, offices.

It is an objective of this invention to provide an air disinfecting device inside an air circulation system of mobile article for carrying people, such as vehicles, trains, MRTs(Mass Rapid Transit), airplanes, or cruise ships.

Accordingly, the invention provides an air disinfecting device, which comprises a duct with a first opening for inlet air flow and a second opening for outlet air flow, a first UVC LED assembly mounted inside the duct, and a power supply providing power to drive the UVC LED assembly. The power supply and the UVC LED could be mounted on one circuit board.

In the air disinfecting device according to one embodiment of the present invention, a material of the duct may be Al, Cu, alloy thereof, or metal-coated plastic.

In the air disinfecting device according to one embodiment of the present invention, an inner surface of the duct may be formed with ARC layer.

In the air disinfecting device according to one embodiment of the present invention, an inner surface of the duct may be a matt surface.

The air disinfecting device according to one embodiment of the present invention may further comprises a photocatalyst layer formed inside an inner surface or both inner surfaces of the duct.

The air disinfecting device according to one embodiment of the present invention may further comprises a second UVC LED assembly mounted inside the duct.

In the air disinfecting device according to one embodiment of the present invention, the first UVC LED assembly may be mounted in a corner of an L-shape of the passage way, or any place within the passage way.

In the air disinfecting device according to one embodiment of the present invention, the passage way may be configured inside an air circulation system of an appliance, a building, or a mobile article for carrying people.

The present invention also provides an air disinfecting device, which comprises a first metal plate and a second metal plate opposite to the first metal plate, a UVC LED assembly mounted on the first metal plate, wherein the second metal plate has an area large enough such that lights emitted from the UVC LED assembly will be enclosed inside the air disinfection device, and a power supply providing power to the UVC LED assembly.

In the air disinfecting device according to one embodiment of the present invention, a surface of the second metal plate facing to the first metal plate may be coated with an anti-reflection layer.

In the air disinfecting device according to one embodiment of the present invention, a surface of the second metal plate facing to the first metal plate may be a first matt surface.

In the air disinfecting device according to one embodiment of the present invention, a surface of the first metal plate facing to the second metal plate may be a second matt surface.

In the air disinfecting device according to one embodiment of the present invention, the first metal plate may include a second concave structure for mounting the UVC LED assembly.

In the air disinfecting device according to one embodiment of the present invention, the second metal plate may include a first concave structure for receiving the lights emitted from the UVC LED assembly.

In the air disinfecting device according to one embodiment of the present invention, a distance between the first metal plate to the second metal plate may be between about 0.1 to 10 cm.

In the air disinfecting device according to one embodiment of the present invention, a distance between the first metal plate to the second metal plate may be between about 0.3 to 1.0 cm.

In the air disinfecting device according to one embodiment of the present invention, the area and the distance may have a ratio ranged about from 1: 1.4 to 1: 40.

In the air disinfecting device according to one embodiment of the present invention, the power supply may be a battery.

The air disinfecting device according to one embodiment of the present invention may further comprise a photocatalyst layer formed inside an inner surface of the second metal plate.

In the air disinfecting device according to one embodiment of the present invention, the first metal plate and the second metal plate may be installed at the air flow outlet on a vent of a vehicle.

In the air disinfecting device according to one embodiment of the present invention, the power supply may be a cigarette lighter or an automobile auxiliary power outlet.

The present invention also provides an air disinfecting device, which comprises a housing with at least one rear opening and at least one front opening such that air flows from the rear opening into the housing and out of the housing through the front opening; a UVC LED assembly inside the housing, for providing UVC lights to disinfect the air flowing inside the housing; and a power supply providing powers to drive the UVC LED assembly.

The air disinfection device according to one embodiment of the present invention may further comprise a first cover with the UVC LED assembly mounted thereinside and a second cover within a concave inner surface to seal the housing.

The air disinfection device according to one embodiment of the present invention may further comprise a lens on the UVC LED assembly such that UVC lights emitted from the UVC LED assembly is collimated toward the second cover.

In one embodiment of the air disinfection device of the present invention, a material of the housing is steel, stainless steel, tinplate, Al, Cu, alloy thereof, or metal-coated plastic, and an inner surface of the housing includes a honeycomb structure, pyramid structure or a screw structure.

In on embodiment of the air disinfection device of the present invention, a material of the concave inner surface of the second cover is UVC absorptive and a material of the inner surface of the housing is UVC absorptive.

The air disinfection device according to one embodiment of the present invention may further comprise a spring clip for clamping on a vent plate on a vent.

The air disinfection device according to one embodiment of the present invention may further comprise two spring clips for clamping on two vent plates on a vent.

In one embodiment of the air disinfection device of the present invention, the two spring clips fasten to a front side of the air disinfection device or a back side of the air disinfection device.

In one embodiment of the air disinfection device of the present invention, the inner surface of the housing is matt, sanded, or imprinted.

In one embodiment of the air disinfection device of the present invention, the power supply includes a battery or a USB port.

In one embodiment of the air disinfection device of the present invention, the power supply is a cigarette lighter or an automobile auxiliary power outlet.

In one embodiment of the air disinfection device of the present invention, the housing comprises an upper plate, a lower plat, and a support therebetween, and the UVC LED assembly is mounted on the support for illuminating the UVC lights to the housing.

The air disinfection device according to one embodiment of the present invention may further comprise a shield for blocking the UVC lights inside the housing without blocking the air flows.

The air disinfection device according to one embodiment of the present invention may further comprise a shield for blocking the UVC lights outside the housing without blocking the air flows.

The present invention also provides an air disinfecting device, which comprises a housing with a rear opening and a front opening such that air flows through the rear opening into the housing and out of the housing through the front opening, wherein a material of the housing is steel, stainless steel, tinplate, Al, Cu, alloy thereof, and an inner surface of the housing is matt, sanded, or imprinted; a UVC LED assembly inside the housing for providing UVC lights to disinfect the air flowing inside the housing; a power supply providing power to drive the UVC LED assembly; and two spring clips, fastened to the housing, for clamping on two vent plates in a vent.

In one embodiment of the air disinfection device of the present invention, a material of an inner surface of the housing is UVC absorptive.

In one embodiment of the air disinfection device of the present invention, the inner surface of the housing includes a honeycomb structure, pyramid structure or a screw structure.

The air disinfection device according to one embodiment of the present invention may further comprise a first cover with the UVC LED assembly mounted thereinside and a second cover within a concave inner surface to seal the housing.

The air disinfection device according to one embodiment of the present invention may further comprise a lens on the UVC LED assembly such that UVC lights emitted from the UVC LED assembly is collimated toward the second cover.

In one embodiment of the air disinfection device of the present invention, the power supply includes a USB port a cigarette lighter or an automobile auxiliary power outlet.

Other advantages of the present invention will become apparent from the following description taken in conjunction with the accompanying drawings wherein are set forth, by way of illustration and example, certain embodiments of the present invention.

### BRIEF DESCRIPTION OF THE DRAWINGS

Further advantages of the present invention may become apparent to those skilled in the art with the benefit of the following detailed description of the preferred embodiments and upon reference to the accompanying drawings in which:
Figure 1 is a schematic top-view illustration of a UVC LED package;
Figure 2 is a schematic cross-sectional illustration of the UVC LED package in Figure 1;
Figure 3 is a schematic cross-sectional illustration of a UVC LED package configured in a portion of a pipeline or duct for disinfecting in according to another embodiment of the present invention;
Figure 4 is a schematic cross-sectional illustration of two UVC LED packages configured in a portion of a pipeline or duct for disinfecting in according to another embodiment of the present invention;
Figure 5 is a schematic oblique illustration of four UVC LED chips configured in a portion of a pipeline or duct for disinfecting in according to another embodiment of the present invention;
Figure 6 is a schematic cross-sectional illustration of one UVC LED assembly configured inside an L-shaped duct for disinfecting in according to another embodiment of the present invention;
Figure 7 is a schematic cross-sectional illustration of a UVC LED package configured between two plates for disinfecting in according to one embodiment of the present invention;
Figure 8 is a schematic oblique illustration of the two plates within a UVC LED package configured between two plates for disinfecting in according to another embodiment of the present invention;
Figure 9 is a schematic oblique illustration of two obstacles sealing two sides of the two plates in according to another embodiment of the present invention;
Figure 9B is a schematic oblique illustration of two extensions covering two sides of the two plates in according to another embodiment of the present invention;
Figures 10A and 10B are schematic oblique illustrations of an air disinfection device applied to a vent of vehicles in according to another embodiment of the present invention;
Figures 11A, 11B, Figure 11C, Figure 11D, Figure 11E, and Figure 11F are schematic cross-sectional illustrations of an air disinfection device applied to a vent of vehicles in according to variant embodiments of the present invention;
Figure 12A is a schematic cross-sectional illustration of a UVC LED package configured between two plates for disinfecting in according to another embodiment of the present invention;
Figure 12B is a schematic cross-sectional illustration of a UVC LED package configured between two plates for disinfecting in according to another embodiment of the present invention;
Figure 12C is a schematic cross-sectional illustration of a UVC LED package configured between two plates for disinfecting in accordance with one embodiment of the present invention;
Figure 13 is a schematic exploded illustration of an air disinfection device applied to a vent of vehicles in according to one embodiment of the prevent invention;
Figures 14A and Figure 14B are schematic illustrations of an air disinfection device applied to a vent of vehicles in according to one embodiment of the prevent invention;
Figure 15 is a schematic cross-sectional illustration of an air disinfection device applied to a vent of vehicles in according to one embodiment of the prevent invention;
Figure 16 is a schematic illustration of an air disinfection device operating with UVC lights inside housing in according to one embodiment of the prevent invention;
Figure 17 a schematic illustration of an air disinfection device applied to a vent of vehicles in according to another embodiment of the prevent invention;
Figure 18A and Figure 18B illustrate shields for blocking UVC lights from the air disinfection device in according to one embodiment of the prevent invention;
Figures 19 illustrate two inner structures of the housing of the air disinfection device in according to one embodiment of the prevent invention;
Figures 20A and 20B illustrate a cross-sectional structure of the housing of the air disinfection device in according to another embodiment of the prevent invention.

### DETAILED DESCRIPTION OF THE INVENTION

Accordingly, while example embodiments of the invention are capable of various modifications and alternative forms, embodiments thereof are shown by way of example in the drawings and will herein be described in detail. It should be understood, however, that there is no intent to limit example embodiments of the invention to the particular forms disclosed, but on the contrary, example embodiments of the invention are to cover all modifications, equivalents, and alternatives falling within the scope of the invention.

In this invention, the term "UVC LED" may refer to a LED chip or an array of LED chips which emits light with wavelength in UVC.

In this invention, the term "UVC LED package" may refer to a UVC LED chip or chips packaged inside a housing or an array of UVC LED packages.

In this invention, the term "UVC LED assembly" may refer to a LED package or an array of LED packages with driving circuits on a board. Power supply, such as battery or power lines, can also be provided in the board.

In this invention, the term "disinfection" may refer to the process of using a disinfectant to destroy, inactivate, or significantly reduce the concentration of pathogenic agents, such as bacteria or viruses.

In this invention, the term "decontamination" may refer to a system or device which can remove or get rid of contamination.

In this invention, the term "sterilization" may refer to the act of sterilizing, such as the rendering of something free from viable microorganisms.

In this invention, the term "passive air flow" may refer to an air flow directly from the environment by fans or air circulation, not by the disinfection device per se.

In this invention, the term "passage way" may refer to a portion or complete of a pipeline or a duct wherein air can be flowed inside the pipeline or duct.

In the drawings, relative dimensions of each component and among every component may be exaggerated for clarity. Within the following description of the drawings the same or like reference numbers refer to the same or like components or entities, and only the differences with respect to the individual embodiments are described.

This invention provides at least one UVC LED package as a source for air sterilization or air disinfection. The UVC LED package is enclosed inside a duct or between a pair of plates. The areas of the metal plates should be large enough to prevent the UVC light leakage. A matt surface or an ARC coating surface inside the duct or metal plates can prevent UVC light from leaking due to the reduced reflection.

The present invention provides an indoor air disinfecting device which can be applied to the air outlet of an air conditioner, a refrigerator, an air purifier, a dehumidifier, or a humidifier. Indoor applications can be more effective due to bacteria, viruses, or foul odors can be irradiated repeatedly by the UVC lights through the air circulation.

The present invention provides an air disinfecting device which can be applied to the vent in the vehicles, airplanes, or cruise ships. For application in car or vehicle, the cigarette lighter, an automobile auxiliary power outlet, or a set of battery can provide power to the UVC LED assembly.

The present invention provides an indoor air disinfection device which can be applied to passage way inside refrigerators, air purifiers, air conditioners, dehumidifiers, or humidifiers.

The present invention provides an air disinfection device which can be applied to the air conditioner in open environments, such as cleanrooms, residential buildings, commercial buildings, hospitals, malls, supermarkets, theaters, stadiums, skating rinks, ice skating rinks, or domes.

For the applications mentioned above, light power or UVC radiation intensity of the UVC LED should match with the airflow for an effective disinfection.

Detailed embodiments of the present invention can be described and shown herein with the drawings.

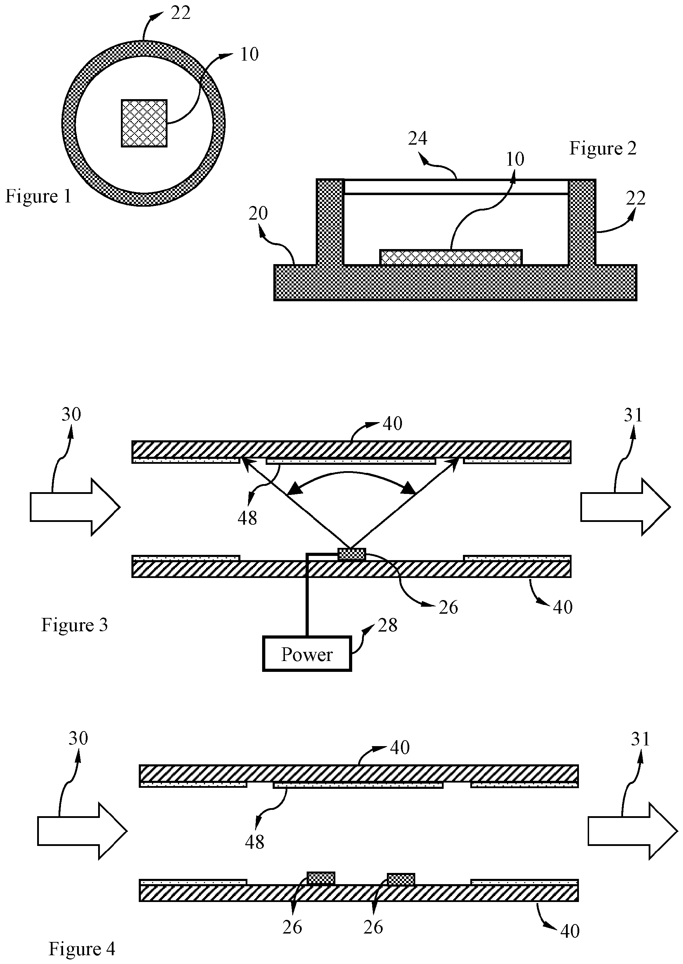

Please refer to Figure 1, wherein a UVC LED package is shown. The UVC LED package includes a UVC LED chip 10 packaged inside a housing 22. Please refer to Figure 2, wherein a cross-sectional view of the UVC LED package is shown. The UVC LED chip 10 is mounted on a substrate 20, which may be a metal or ceramic substrate with circuits thereon. The ceramic or metal housing 22 is made firmly on the substrate 20. A cap 24 is hermetically sealed with the housing 22, and UVC lights emitted from the UVC LED chip 10 can pass through the cap 24. The material of the cap 24 can be quartz. Wavelength emitted by the UVC LED chip 10 is ranged from 200-280nm.

The emitting angle of the UVC LED package ranges from 120°-140° if the cap 24 is flat, and may be 60°-140° if the cap 24 is designed with an optical component. The emitting angle is large enough to cover a relatively large area for sterilization.

Please refer to Figure 3, wherein a cross-sectional view of an air disinfection device of one embodiment is shown. At least one UVC LED assembly 26 is configured inside of a duct 40 within the passage way, such as a portion of a pipeline or a duct, and powered by a power supply 28. The duct 40 may have a square cross-sectional shape, a circular shape or other shape. The duct 40 is made by materials unlikely to be degraded by UVC irradiation, such as metal. For example, Al, Cu, tinplate, steel, stainless steel tempered, alloy thereof, and metal-coated plastic can be suitable for the duct 40. Note that the conventional pure plastic is not suitable for the duct 40. The area 48 inside the duct 40 illuminated by the UVC lights can be coated with silver or other types of nano particles for enhancing the photocatalyst reaction. In this embodiment, the foul odor can be eliminated by the photocatalyst function. Other types of photocatalyst such as Titanium Oxide, Gallium Phosphide, and Gallium Arsenide can also be applied. In one embodiment, other surfaces inside the duct 40 can be coated with a photocatalyst layer.

The power supply 28 can be a battery, mains electricity, utility power, power grid, domestic power, wall power, household power, household electricity, house current, powerline, line power, AC power, city power, or street power. In one embodiment, power supply 28 should provide enough power to the UVC LED such that irradiation of the UVC LED can match the air flow for an effective disinfection. For example, if the air flow is large, UVC LED should provide more light density to disinfect or sterilize bacteria or viruses. Thus, the power supply 28 should provide enough electric power to the LED assembly so as to have enough irradiation for an effective sterilization, or more UVC LED chips or UVC LED packages have to be used. In order to clarify the embodiments of the present invention, power for the UVC LED package will not be shown in the following drawings.

Passive air flow 30 from inlet to outlet of the passage way is illuminated by the UVC LED 26. In the present invention, the air flow 30 driven into the inlet of the passage way is provided by the environment, such as the cases of refrigerators, air purifiers, air conditioners, dehumidifiers, or humidifiers of appliances, air circulation system in buildings for habitat or living, such as residential houses, wards in hospitals, theaters, cleanrooms, factories, offices, or air circulation system in mobile articles for carrying people, such as vehicles, trains, MRT, airplanes, or cruise ships. The air 31 then flows out of the outlet of the passage way. All these appliances provide air flow and/or local air circulation. For example, if the UVC LED assembly is configured within the passage way of the refrigerator or other appliances, the UVC lights will sterilize bacteria or viruses when they pass through the air disinfection device. Even when the light density or illumination of UVC light emitted by the UVC LED assembly is not high enough to kill all bacteria or viruses at one pass, the air circulation inside the refrigerator will bring them back to the air disinfection device again and again, for repeated sterilization.

For the air disinfection device of the present invention applied to the air purifiers, air conditioners, dehumidifiers, or humidifiers, the air circulation inside the residential houses, hospitals, or commercial buildings have similar function as that inside the refrigerator. Thus, the air circulation configuration would be better for repeated disinfection. The invention can also be applied to airplanes, cruise ships, trains, MRT or vehicles.

The air disinfection device of the present invention can also be applied to the air circulation system of the clean room in a factory, office, theater, mall, or stadium.

The air disinfection device of the present invention can be applied to the air conditioners in an open environment, such as stations, hospitals, commercial buildings, airports, piers, train and subway stations, stadiums, or malls. The dosage of the UVC can be designed such that bacteria and viruses are killed completely. This is important for the situations like biological terrorism, SARS, COVID 19, sick buildings, cruise ship disease outbreaks, toxic molds, and epidemics of asthma and allergies.

The air disinfection device of the present invention can include more than one UVC LED packages, as shown in Figure 4. Two UVC LED assembly 26 are mounted inside the metal duct 40 if the air flow is larger. High light density in the duct 40 is provided to match the air flow, and hence the bacteria, or viruses can also be eliminated in one pass.

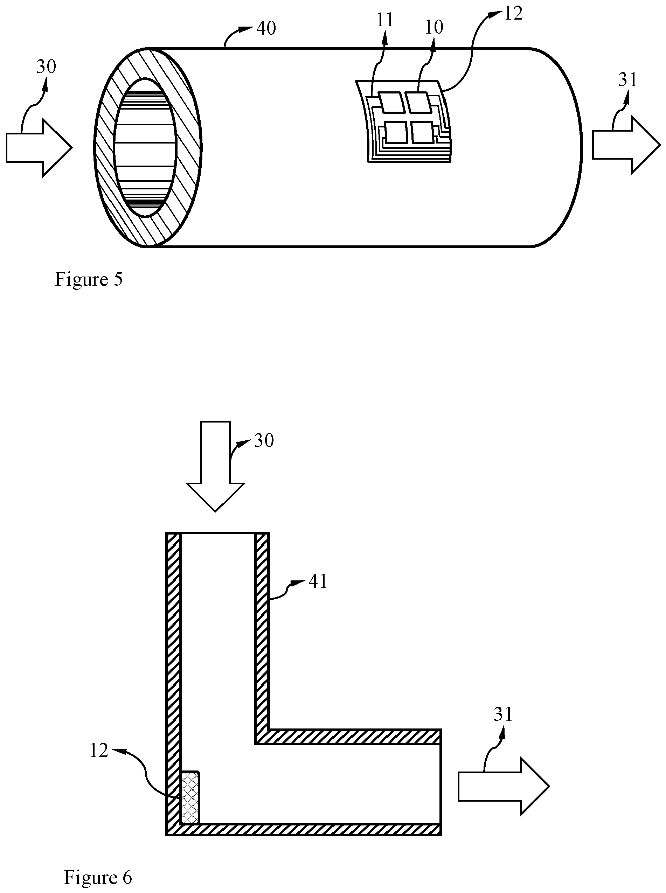

Please refer to Figure 5, wherein an oblique view of the disinfection device configured in a pipe or duct 40. In the embodiment, four UVC LED packages 10 as an array are packaged on an assembly 12 with circuits 11 for an effective and complete sterilization. A portion of the duct wall is designed such that the UVC LED assembly 12 can be installed. The present invention is also suitable for an L-shaped duct 41, as shown in Figure 6, or other shapes(not shown in drawings) within a duct. In some applications, air flow at the corner of the L-shape duct forms turbulence, and it becomes a good place to install a disinfection device. Generally, UVC LED assembly can be configured within any place of a duct, and it would be preferred if UVC LED chips are configured before or after filters in a passage way.

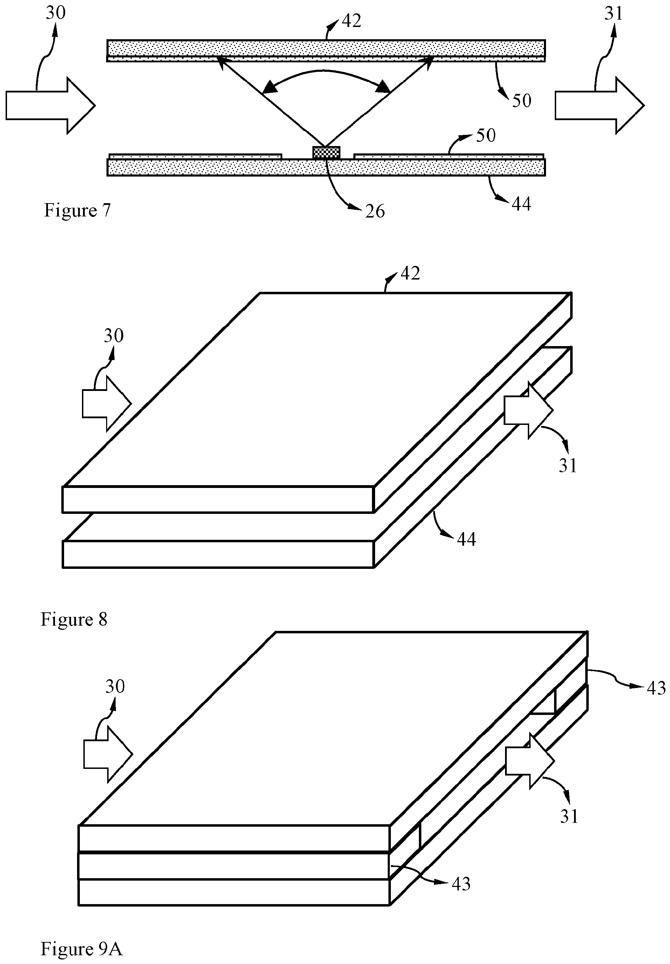

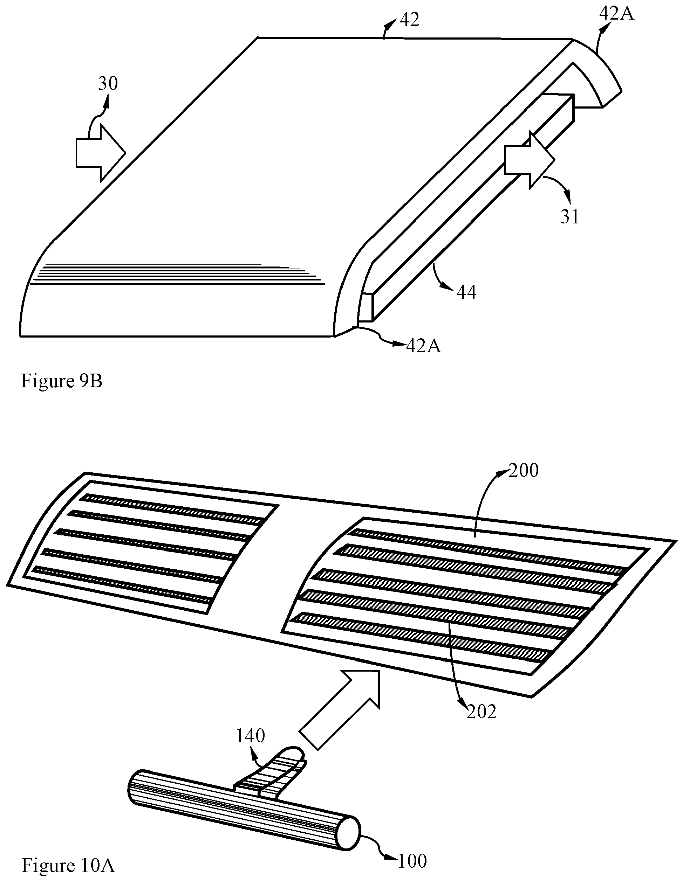

Please refer to Figure 7 and Figure 8, wherein another embodiment of the present invention is shown. A disinfection device includes an upper plate 42 and a lower plate 44 which form two sides of a housing. The air flow 30 is pushed through the inlet of the housing and the air flow 31 is pushed out of the outlet of the housing. The other two sides of the housing, can be open (Figure. 8), sealed (Figure 9A) or covered by extensions of a plate or both plates (Figure 9B). For example, two posts 43 or extensions 42A of the upper plate 42 can cover the other two sides of the housing, such that UV lights inside the housing can be contained as shown in Figure 9A and Figure 9B respectively. The UVC LED assembly 26 is mounted on the lower plate 44 and the power supply for the UVC LED assembly 26 is not shown in Figure 7. Materials of the plates 42 and 44 can be Al, Cu, SST, tin, tinplate, steel, alloy thereof, and metal coated plastic. Areas of the two metal plates are large enough to prevent the UVC light leakage. In the present invention, the embodiment shown in Figure 9B is preferred.

An anti-reflecting coating(ARC) layer 50 is formed inside the plates 42 and 44 such that reflected UVC lights can be substantially reduced or eliminated. As example of UV ARC can refer to US patent, US9,488,852. Another way to form anti-reflect surface is to form matt surfaces on the inner surfaces of the plates. The purpose of forming the ARC layers or matt surfaces is to reduce the possibility of UVC light leakage. When reflections of the UVC light are reduced, UVC leakage can be prevented.

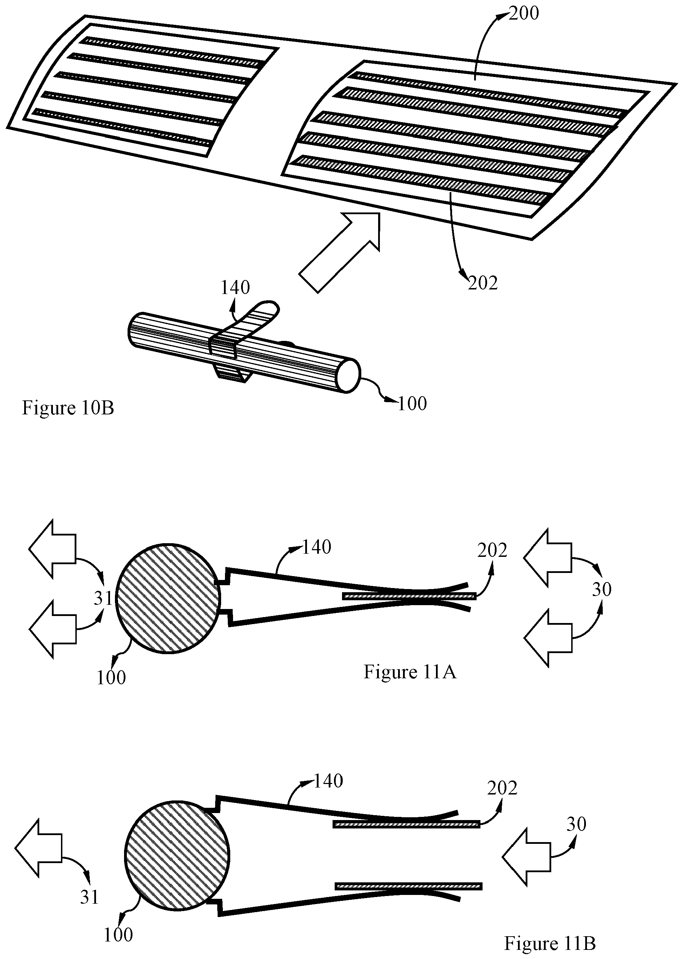

The distance between the upper plate 42 and the lower plate 44 can range from 0.1 - 10 cm for mobile device applications, and preferred from 0.3 - 1.0 cm. The ratio between the distance and the area of one plate is ranged about from 1:1.4 to 1:40. The purpose of the distance and the ratio is to make sure that the UVC lights emit from the lower plate 44 and are reflected and absorbed by the plates 42 and 44 only and do not leak out of the housing. In this embodiment, the air disinfection device 100 can be applied to vehicles, for example, placed (inserted into or hung onto) at air condition vents 200 of cars, as shown in Figures 10. Two spring clips 140 may sandwich or clamp on a vent plate 202 as shown in Figure 10A, or two vent plates 202 as shown in Figure 10B. Please notice that front and rear openings for air flow through are not shown in these figures. Thus, the air flow 30 is supplied by fan (blower) of the car. In addition to use a battery as the power, the cigarette lighter or automobile auxiliary power outlet can be used to provide electric power to the UVC LED assembly.

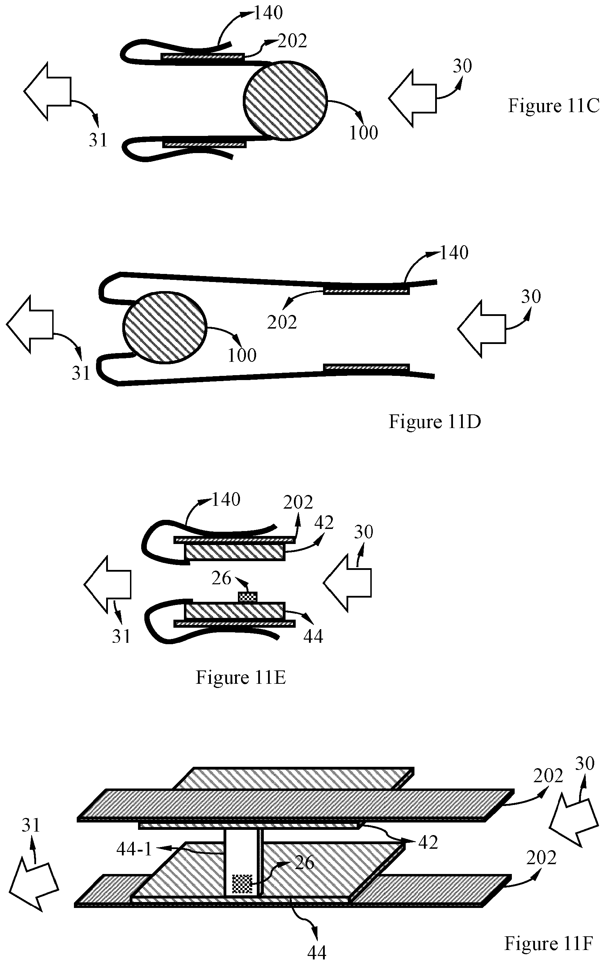

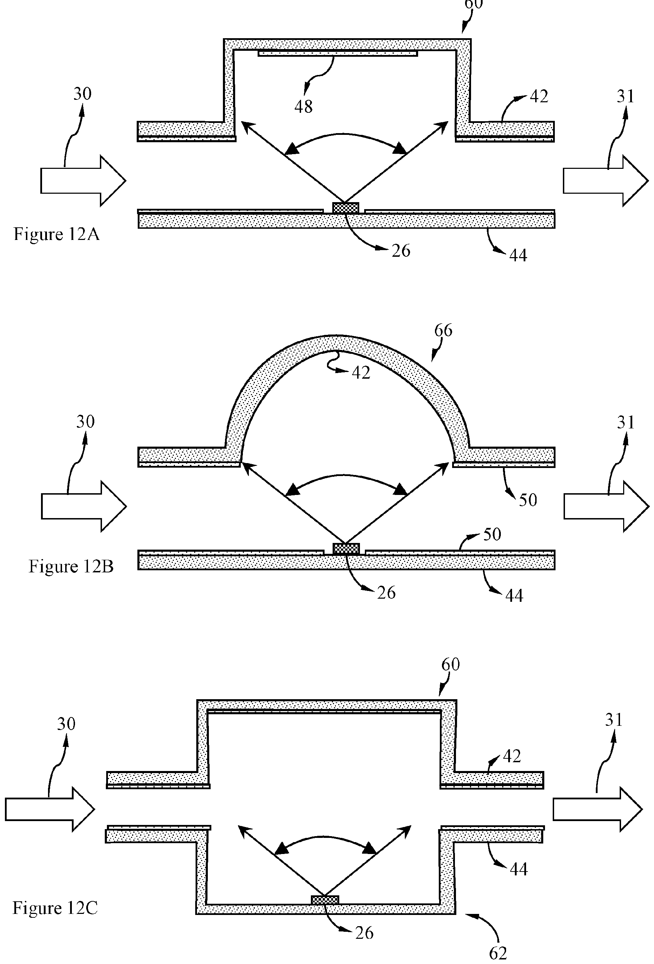

Some variant embodiments of cross-section illustrations of the air disinfection device 100 installed on the vent plates 202 are shown in Figure 11A, 11B, 11C, 11D, 11E, and 11F. In Figure 11A, two spring clips 140 are fastened to the back side of the air disinfection device 100 body, and clamp on a vent plate 202. The air flow 30, above and below the vent plate 202, blows from the back side of the air disinfection device 100, and the air flow 31 is purged out of the front side of the air disinfection device 100. In Figure 11B, two spring clips 140 are also fastened to the back side of the air disinfection device 100 body, and clamp on two vent plates 202 respectively. Thus, the air flow 30 between the two vent plates 202 blows the bask side of the air disinfection device 100 and the air flow 31 is purged out of the front side of the air disinfection device 100. In Figure 11C, two spring clips 140 are fastened on the front side of the air disinfection device 100 body, and clamp on two vent plates 202 respectively. In this embodiment, the air disinfection device 100 can be installed between two vent plates 202 and inside the vent 200. An embodiment as shown in Figure 11D, two spring clips 140 are fastened on the front side of the air disinfection device 100 body, and clamp on two vent plates 202 respectively. The air disinfection device 100 also is installed between two vent plates 202 but outside the vent 200. For the embodiments shown in Figures 7, 8, 9, and 12, the air disinfection device 100 with an upper plate 42 and a lower plate 44 can be installed between two vent plates 202 and inside the vent 200, as shown in Figure 11E. The UVC LED assembly 26 can be mounted on the lower plate 44 as shown in the drawings, but can also be mounted on the upper plate 42. In this embodiment, the two posts 43 in Figure 9A can be applied to this embodiment. For the air disinfection device illustrated in the Figures 12, can also be applied to the embodiment in the Figure 11. In the above four embodiments, body of the air disinfection device 100 has a circular cross-sectional shape. For the embodiments shown in Figures 7, 8, and 9, the two spring clips 140 can be fastened to the upper plate 42 and lower plater 44 respectively as shown in Figure 11E, and the UVC LED 26 is mounted on the lower plate 44 or upper plate 42. The UVC LED 26 can be mounted on a support 44-1 as shown in Figure 11F. Similar to the embodiment shown in Figure 11E, the upper plate 42 and the lower plate 44 are configures between two vent plates 202. In this embodiment, the two spring clips 140 are not shown in Figure 11F for the clarity. The UVC LED 26 is mounted on the backside of the support 44-1 to illuminate a direction where the air flow 30 comes from. The support 44-1 also provides means for fastening the upper plate 42 and the lower plate 44.

Some structures can be made to avoid the UVC light bounced out of the housing. Please refer to Figure 12A, wherein the upper plate 42 has a square concave structure 60 such that the UVC light emitted from the UVC LED package 26 is reflected within the square concave structure 60. A photocatalyst layer 48 can be formed inside the square concave structure 60.

Another embodiment of the air disinfection device can be referred to Figure 12B. The concave structure 66 on the upper plate 42 is a dome-like structure, wherein UVC light is reflected inside the concave structure 66. The photocatalyst layer 48 in Figure 12A can be formed in the concave structures 66 in Figure 12B. If necessary, the low plater 44 can be designed another concave structure, thus the reflected UVC light is confined within the space between the upper concave structure 60 of the upper plate 42 and lower concave structure 62 of the lower plate 44, as shown in Figure 12C. In this embodiment, the UVC LED assembly 26 is mounted inside the lower concave structure 62. In the above embodiments, the concave structure either in the upper plate 42 or lower plate 44 can be of other shapes, such as concave structure 60 in Figure 12A may extend to a direction perpendicular to the air flow. Thus, the concave structure 60 in Figure 12A is not a square shape. Similarly, the concave structure 66 in Figure 12B may also extend to the direction and hence constitutes a partial cylindrical shape in top view. The photocatalyst layer 48 in Figure 12A can be formed in the concave structures 60 and 62 in Figure 12C.

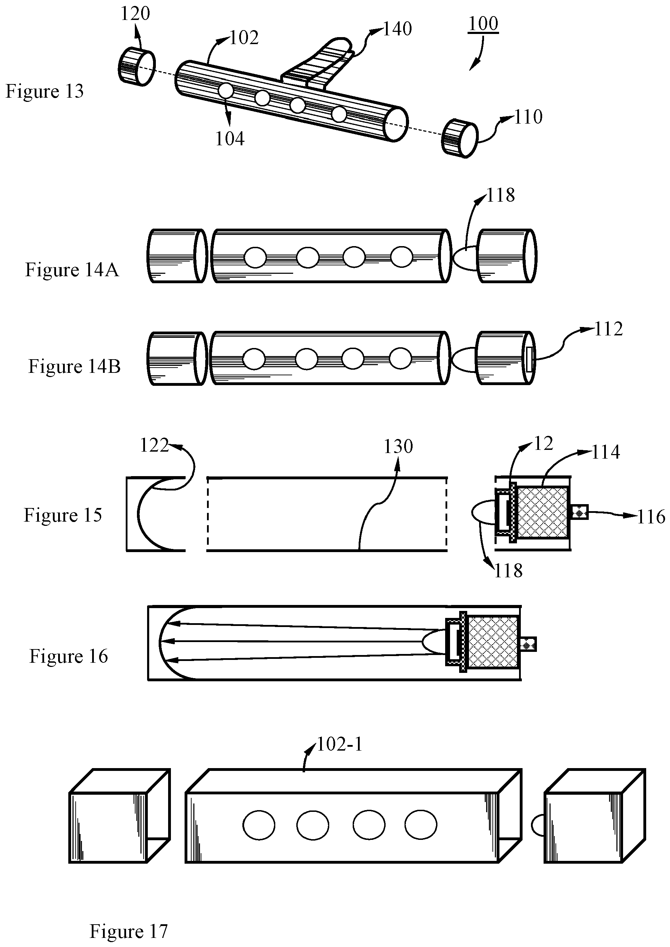

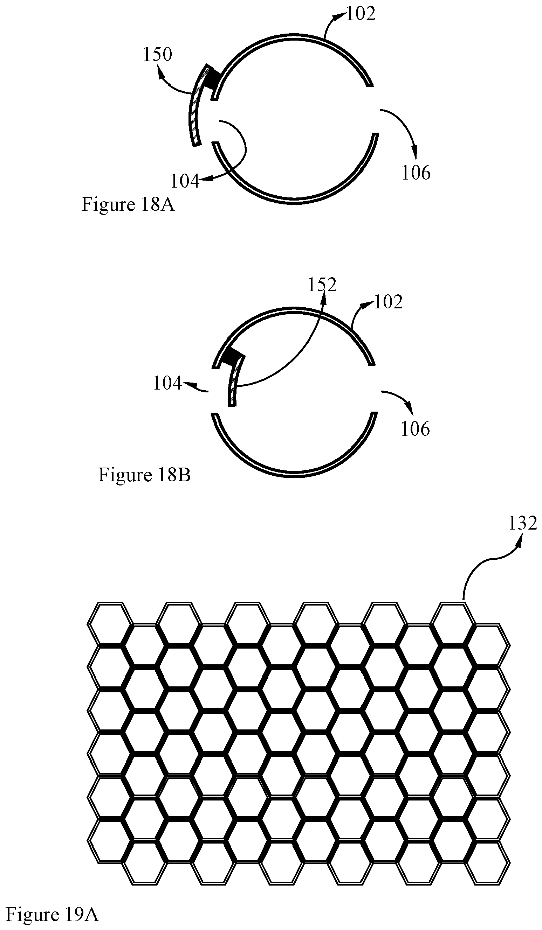

For one embodiment in the present invention, detail structures of the air disinfection device 100 are described. Please refer to Figure 13 and Figures 14, wherein the air disinfection device 100 includes a housing 102 with at least one rear opening and at least one front opening. Either rear or front opening 104 can be one large opening or several small openings, and four front openings are shown in this embodiment. A first cover 110 and a second cover 120 fasten the housing 102 to provide an environment for air to flow through inside of the housing 102, such that the air flow is illuminated by UV lights. Materials of the housing 102, first cover 110 and second cover 120 can be any metal, such as steel, stainless steel, tinplate, aluminum, iron or steel, or compound of any metal. Several front openings 104 allow air to flow out of the housing 102, while rear openings 106 which can be referred to Figures 18 provide air to flow into the housing 102. A spring clip 140 clamps sheets on the vent in Figures 10. A UVC LED package is mounted inside the first cover 110 with an optional lens 118, and a battery to power the UVC LED. In one embodiment, a socket 112, such as USB port, provides an interface for powering the UVC LED.

Please refer to Figure 15 and Figure 16, wherein inside structures of the air disinfection device are shown. The first cover 110 includes a UVC LED package 12, a lens 118 for shaping UVC light from the UVC LED package 12, a battery 114 for powering the UVC LED package 12, and a switch 116 for turning the UVC LED on/off. Another visible LED, which is not shown in the drawings, can be optionally mounted on the first cover 110 for indicating whether the battery is workable or the UVC LED is out of service. The second cover 120 optionally includes an inner concave surface 122 such that UVC light irradiated from the UVC LED package 12 on the inner surface 122 can be absorbed easily.

When the first cover 110 and the second cover 120 sealed with the housing 102, most UVC lights emitted from the UVC LED package 102 will irradiate to the inner concave surface 122, because the optional lens 118 shapes the UVC lights from the UVC LED package 12 into a small emitting angle. Small emitting angle of the UVC LED package 12 is a preferred embodiment. A shape of the inner surface 122 is recess or concave so that UVC light can be reflected inside the inner surface 122. A preferred shape of the inner surface 122 can be semi-sphere or semi-ellipsoid.

Outline of the housing 102 in the above embodiment is cylindrical. However, another shape can be applied to this invention. A square columnar housing 102-1 is shown in Figure 17. In fact, cross-sectional shapes of the housing 102, although not in in the drawings, may include eclipse, triangle, square, rectangular, polygon, or other shapes.

In order to shield or block the UVC lights from the housing 102 outside the front openings 104, an outer shield 150 or inner shield 152 can be applied to the housing 102, as shown in Figures 18. The air flow from the vent is blown into the housing 102 via the rear openings 160. Then the air flow is irradiated by the UVC lights emitted from the UVC LED package, and then flow out of the housing 102 through the front openings 104. The outer shelter 150, covering the front openings 104 such that UVC lights inside the housing 102 can be blocked, still lets air flow move out. An inner shelter 152 can also be applied inside the housing 102.

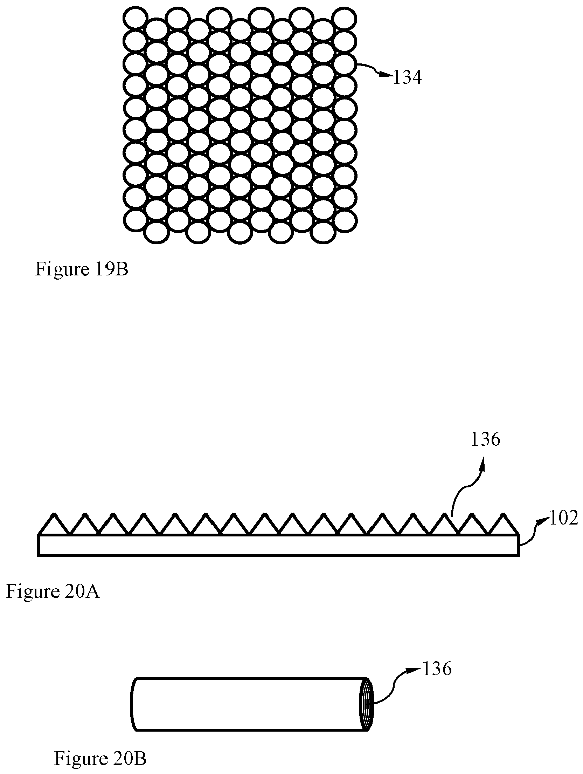

The inner surface 130 of the housing 102 may optionally include regular or irregular rough structures or may be matte to reduce reflectiveness of the inner surface 130. In one embodiment, the inner surface 130 of the housing 102 may also be sanded or imprinted. Moreover, the inner surface 130 may include a honeycomb structure 132 or another honeycomb structure 134 as shown in Figure 19A and Figure 19B, for antireflecting the UVC lights inside the housing 102. Features of the honeycomb structures 132 and 134 can be optimized for absorbing the UVC lights emitted from the UVC LED package 12. Moreover, the inner surface 130 and inner concave surface 122 may be coated with UVC light absorption materials, such as Avain Black-S^{®}, silicon oxide coatings, or magnesium oxide coatings. The materials of the honeycomb structures 132 and 134 can be the UVC lights absorption materials or metal similar to the housing 120. A simple structure of the inner surface 130 can be a screw structure 136, as shown in Figure 20A and Figure 20B. Materials of the screw structure 136 can be UVC lights absorption materials or the same to the housing 120. The inner surface of the housing 102 may include a pyramid structure which is not shown in the figures.

In the present invention, UVC LED is provided for disinfection, which consumes low power and is compact compared to a conventional UV light bulb or tube.

In the present invention, the air disinfection device is designed within a passive air flow and hence the power consumption can be even lower. Thus, the air disinfection device of this invention is placed in an environment with air flow.

In the present invention, large enough metal plates as air disinfection housing are provided to prevent UVC light leakage. Further, ARC layer or matt surfaces can be provided to decrease the UVC light leakage.

Although the present invention has been described in accordance with the embodiments shown, one of ordinary skill in the art will readily recognize that there could be variations to the embodiments and those variations would be within the spirit and scope of the present invention. Accordingly, many modifications may be made by one of ordinary skill in the art without departing from the spirit and scope of the appended claims.

## Claims

1. An air disinfecting device, comprising:
a housing with a rear opening and a front opening such that air flows through the rear opening into the housing and out of the housing through the front opening;
a UVC LED assembly inside the housing for providing UVC lights to disinfect the air flowing inside the housing; and
a power supply providing power to drive the UVC LED assembly.

2. The air disinfection device according to claim 1, further comprising a first cover with the UVC LED assembly mounted thereinside and a second cover within a concave inner surface to seal the housing.

3. The air disinfection device according to claim 2, further comprising a lens on the UVC LED assembly such that UVC lights emitted from the UVC LED assembly is collimated toward the second cover.

4. The air disinfection device according to claim 2, wherein a material of the housing is steel, stainless steel, tinplate, Al, Cu, alloy thereof, or metal-coated plastic, and an inner surface of the housing includes a honeycomb structure, pyramid structure or a screw structure.

5. The air disinfection device according to claim 4, wherein a material of the concave inner surface of the second cover is UVC absorptive and a material of the inner surface of the housing is UVC absorptive.

6. The air disinfection device according to claim 1, further comprising a spring clip for clamping on a vent plate on a vent.

7. The air disinfection device according to claim 1, further comprising two spring clips for clamping on two vent plates on a vent.

8. The air disinfection device according to claim 7, wherein the two spring clips fasten to a front side of the air disinfection device or a back side of the air disinfection device.

9. The air disinfection device according to claim 5, wherein the inner surface of the housing is matt, sanded, or imprinted.

10. The air disinfection device according to claim 2, wherein the power supply includes a battery or a USB port.

11. The air disinfection device according to claim 2, wherein the power supply is a cigarette lighter or an automobile auxiliary power outlet.

12. The air disinfection device according to claim 1, wherein the housing comprises an upper plate, a lower plat, and a support therebetween, and the UVC LED assembly is mounted on the support for illuminating the UVC lights to the housing.

13. The air disinfection device according to claim 2, further comprising a shield for blocking the UVC lights inside the housing without blocking the air flows.

14. The air disinfection device according to claim 2, further comprising a shield for blocking the UVC lights outside the housing without blocking the air flows.

15. An air disinfecting device, comprising:
a housing with a rear opening and a front opening such that air flows through the rear opening into the housing and out of the housing through the front opening, wherein a material of the housing is steel, stainless steel, tinplate, Al, Cu, alloy thereof, and an inner surface of the housing is matt, sanded, or imprinted;
a UVC LED assembly inside the housing for providing UVC lights to disinfect the air flowing inside the housing;
a power supply providing power to drive the UVC LED assembly; and
two spring clips, fastened to the housing, for clamping on two vent plates in a vent.

16. The air disinfection device according to claim 15, wherein a material of an inner surface of the housing is UVC absorptive.

17. The air disinfection device according to claim 16, wherein the inner surface of the housing includes a honeycomb structure, pyramid structure or a screw structure.

18. The air disinfection device according to claim 15, further comprising a first cover with the UVC LED assembly mounted thereinside and a second cover within a concave inner surface to seal the housing.

19. The air disinfection device according to claim 18, further comprising a lens on the UVC LED assembly such that UVC lights emitted from the UVC LED assembly is collimated toward the second cover.

20. The air disinfection device according to claim 15, wherein the power supply includes a USB port a cigarette lighter or an automobile auxiliary power outlet.
